# DEMANDE DE BREVET EUROPEEN

(11) **EP 2 158 943 A2**
(43) Date de publication de la demande: **03.03.2010**
(21) Numéro de dépôt: 09161939.5
(22) Date de dépôt: 24.08.2006
(51) Int. Cl.: A61Q 5/10, A61Q 5/04, A61Q 5/06, A61Q 5/08, A61K 8/04, A61K 8/37, A61K 8/22, A61K 8/23, A61K 8/81, A61K 8/87, A61K 8/88, A61K 8/85, A61K 8/41

(54) **Composition oxydante comprenant des composés insolubles, procédés mettant en oeuvre cette composition**

(30) Priorité: 25.08.2005 FR 0508754
(62) Demande divisionnaire de: 06291348.8
(71) Demandeur: L'Oréal, 75008 Paris (FR)
(72) Inventeur: Kravtchenko, Sylvain, 200021, Shangai (CN); Dubief, Claude, 78150, Le Chesnay (FR)
(74) Mandataire: Dossmann, Gérard

(57) **Abrégé**

- on laisse reposer la chevelure sur laquelle a été appliquée la composition oxydante, puis
- on rince par une composition aqueuse.

15. Utilisation de la composition oxydante prête telle que définie à l'une quelconque des revendications 1 à 9 pour la teinture ou pour la déformation permanente ou pour la décoloration ou pour l'éclaircissement ou pour la coloration des fibres kératiniques en particulier de fibres kératiniques humaines.

16. Dispositif à 2 compartiments pour la teinture ou pour la déformation permanente ou pour la décoloration des fibres kératiniques en particulier de fibres kératiniques humaines comprenant un premier compartiment renfermant soit une composition de coloration d'oxydation ou directe, soit une composition réductrice, soit une première composition oxydante contenant de préférence un sel peroxygéné, et un second compartiment renfermant la composition oxydante prête telle que définie à l'une quelconque des revendications 1 à 9.

L'invention porte sur une composition oxydante prête à l'emploi destinée au traitement des matières kératiniques, telles que les fibres kératiniques humaines, en particulier les cheveux, comprenant dans un milieu cosmétiquement acceptable :
(a) au moins un agent oxydant,
(b) au moins 30% en poids d'au moins un composé organique oxygéné non colorant particulier insoluble dans l'eau, par rapport au poids total de ladite composition, et
(c) au moins 20% en poids d'eau par rapport au poids total de ladite composition,

Et sur des procédé de décoloration, de décapage, de teinture, d'éclaircissement ou de déformation permanente des fibres kératiniques humaines et en particulier des cheveux, ainsi que l'utilisation de ladite composition pour la décoloration, le décapage, la teinture, l'éclaircissement et la déformation permanente des fibres kératiniques humaines et en particulier des cheveux.

## Description

La présente invention a trait à des compositions oxydantes prêtes à l'emploi destinées au traitement des matières kératiniques comprenant un composé insoluble dans l'eau, à des procédé de décoloration ou de décapage, de teinture, d'éclaircissement ou de déformation permanente des fibres kératiniques humaines et en particulier des cheveux et à l'utilisation de ladite composition pour la décoloration, le décapage, la teinture, l'éclaircissement ou la déformation permanente desdites fibres.

Les compositions oxydantes sont utilisées pour éclaircir ou décolorer la fibre kératinique. En effet, la décoloration ou l'éclaircissement des cheveux est obtenu par oxydation des pigments de mélanine, cette oxydation pouvant conduire à l'extrême, à une solubilisation et une élimination totale de ces pigments.

Le but de la décoloration est généralement double. Il peut s'agir soit simplement de donner aux cheveux un aspect plus clair, soit de les préparer à l'application d'un produit de coloration (direct ou d'oxydation), la nuance résultante étant en général plus claire que la nuance naturelle.

Il est connu de décolorer les cheveux à l'aide de pâtes (ou cataplasmes) appliquées directement sur les cheveux et obtenues en mélangeant, au moment de l'emploi, une composition décolorante à base de dérivés peroxydés (agents oxydants) avec de l'eau ou plus habituellement encore avec de l'eau oxygénée. La composition décolorante est, de façon connue, constituée d'un dérivé peroxydé, généralement un persulfate ou un perborate de sodium, de potassium ou d'ammonium et parfois un sel d'acide percarboxylique ou un peroxyde, par exemple de baryum, de strontium, d'urée ou de mélanine. Le plus souvent, ces compositions contiennent en outre, de façon connue, des agents fortement alcalins, tels que des métasilicates, des phosphates ou des carbonates alcalins ou alcalino-terreux (régulateurs de pH). Enfin, elles peuvent par ailleurs éventuellement contenir d'autres additifs ou adjuvants classiques dans le domaine : agents de contrôle du dégagement d'oxygène dans le mélange avec l'eau oxygénée, des épaississants, tels que des dérivés de cellulose (carboxyméthylcellulose par exemple) ou l'amidon et ses dérivés, ou bien encore les gommes de guar, de xanthane et les alginates ; des agents tensio-actifs, en particulier anioniques (alkysulfates notamment) ; des séquestrants ; des parfums. De telles compositions décolorantes sont décrites par exemple, dans « The Science of Hair Car » par C. Zviak, Marcel Decker Inc. 1986, pages 225-226.

Les compositions décolorantes peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.

Le processus de décoloration de la fibre dure généralement entre 20 minutes et 1 heure. Ce temps de pose varie selon la forme de la composition oxydante (crème, shampooing, poudre, solution ou émulsion), selon le niveau ou le degré d'éclaircissement souhaité, et selon la concentration des réactifs de la composition oxydante prête à l'emploi, c'est-à-dire la quantité de composés alcalins, de réactifs peroxygénés, le volume d'eau oxygénée ou la proportion de poudres renforçatrices.

On cherche depuis longtemps à accélérer le procédé de décoloration ou d'éclaircissement de la fibre kératinique et à augmenter le degré d'éclaircissement de la fibre, c'est-à-dire à éclaircir plus.

Dans le but de diminuer le temps de pose, il serait tentant d'augmenter la proportion des réactifs au sein de la composition oxydante. Or une concentration plus importante des composants conduirait inévitablement à une altération plus importante de la fibre kératinique, voire à une irritation ou une dermite du cuir chevelu ou l'apparition de picotements.

Les compositions d'oxydation sont également utilisées dans les procédés de déformation permanente, en tant que composition fixante permettant la reconstruction de la fibre dans la forme souhaitée.

Au cours d'un procédé de déformation permanente des cheveux, les cheveux sont enroulés autour de bigoudis, ils sont ensuite imprégnés d'une composition réductrice, dite frisante, que l'on laisse poser entre 5 minutes et 1 heure selon la nature du cheveu et selon le réducteur employé. La forme du cheveu est ensuite fixée à l'aide d'une composition oxydante, qui est appliquée pendant environ 5 minutes, puis laissée poser pendant environ 5 à 30 minutes. Après déroulage des mèches, la composition oxydante est appliquée sur les pointes pendant environ 5 minutes, puis est laissée poser pendant environ 5 minutes. Enfin, la chevelure ainsi traitée est rincée. Ce procédé classique de déformation permanente est long et fastidieux.

Comme pour le procédé de décoloration, il serait souhaitable de mettre au point une composition oxydante plus efficace conduisant à un temps de pose plus court, et diminuant ainsi la durée du procédé de déformation permanente.

Il existe donc un besoin de compositions oxydantes pour la décoloration ou pour la déformation permanente plus efficaces, c'est-à-dire réagissant plus rapidement avec la structure de la fibre, tout en conservant une bonne innocuité vis-à-vis de ladite fibre et du cuir chevelu.

Dans le cadre de la coloration d'oxydation, on procède généralement au mélange au moment de l'emploi d'une composition contenant les colorants d'oxydation habituellement à un pH alcalin, avec une composition oxydante généralement à base d'eau oxygénée formulée à pH acide. Le mélange obtenu est appliqué sur la tête avec des temps de pose pouvant aller jusqu'à une heure. Dans le cas de la coloration indirecte éclaircissante le ou les colorants d'oxydation sont remplacés par au moins un colorant direct.

Il existe un besoin d'accélérer le processus de coloration et/ou de diminuer la concentration en eau oxygénée pour limiter les altérations des fibres kératiniques.

Or, après d'importantes recherches menées sur la question, la demanderesse vient maintenant de découvrir de manière surprenante qu'il est possible d'obtenir des compositions oxydantes prêtes à l'emploi plus efficaces et ayant une bonne innocuité, tout en conservant les proportions des réactifs des compositions oxydantes usuelles. La demanderesse a ainsi mis au point des compositions oxydantes prêtes à l'emploi ne présentant pas les inconvénients précités.

L'invention concerne également les procédés de décoloration, de décapage, de teinture d'oxydation, d'éclaircissement et des procédés de déformation permanente des fibres kératiniques, l'utilisation de ladite composition pour la décoloration, le décapage, la teinture, l'éclaircissement ou la déformation permanente desdites fibres ainsi que des dispositifs de teinture ou « kits » à plusieurs compartiments.

D'autres caractéristiques, aspects, objets et avantages de l'invention figurant dans la description ci-dessous permettront de mieux cerner l'invention.

La présente invention a donc pour objet une composition oxydante prête à l'emploi destinée au traitement des matières kératiniques, telles que les fibres kératiniques humaines, en particulier les cheveux, comprenant dans un milieu cosmétiquement acceptable :
(a) au moins un agent oxydant,
(b) au moins 30% en poids d'au moins un composé organique oxygéné non colorant insoluble dans l'eau par rapport au poids total de ladite composition particulier, et
(c) au moins 20% en poids d'eau par rapport au poids total de ladite composition.

L'agent oxydant est choisi de préférence parmi le peroxyde d'hydrogène et les composés susceptibles de produire par hydrolyse du peroxyde d'hydrogène tels que le peroxyde d'urée, les bromates de métaux alcalins, les persels tels que les perborates, persulfates, percarbonates, et peroxydes de métaux alcalins ou alcalino-terreux, comme le sodium, le potassium, le magnésium, les peracides ou leurs mélanges ; et les enzymes oxydases parmi lesquelles on peut citer les peroxydases, les oxydo-réductases à 2 électrons telles que les uricases et les oxygénases à 4 électrons comme les laccases, le cas échéant en présence de leur substrat approprié. Le peroxyde d'hydrogène est particulièrement préféré.

La teneur en agent oxydant dans la composition prête à l'emploi est comprise entre 2 et 35 % en poids de la composition prête à l'emploi, de préférence entre 5 et 30 % en poids de la composition prête à l'emploi.

Par composé organique oxygéné, on entend, au sens de la présente invention, tout composé organique comportant au moins un atome d'oxygène dans sa structure moléculaire élémentaire.

Par insoluble dans l'eau selon l'invention, on entend des composés présentant une solubilité dans l'eau à température ambiante (25°C) inférieure à 0,5 % en poids.

Les composés organiques oxygénés de l'invention peuvent être des composés polymériques ou non polymériques.

Les composés organiques oxygénés insolubles polymériques selon l'invention sont de préférence choisis parmi les polyamides 6, 66, 11, les polyesters, les polyuréthanes, les polycyanoacrylates, les polyméthacrylates de méthyle, les polycarbonates, le téflon, les résines ou élastomères de silicone.

Les composés organiques oxygénés non polymériques de l'invention peuvent être notamment choisis parmi les alcools gras, les esters, amides, éthers d'acides ou d'alcools. Parmi les esters, amides et éthers d'acides ou d'alcools gras, on peut citer les chaînes grasses des acides ou alcools gras comportant de 8 à 40 atomes de carbone et étant éventuellement hydroxylés. Parmi les alcools gras, on peut citer les alcools gras comportent de 8 à 40 atomes de carbone. A titre d'exemple, citons : le mono et le di-stéarate d'éthylène glycol, le monooléate de pentaerythritol, le tristéarate de sorbitan, le dioléate de glycérol, les esters, amides et éthers gras d'éthylène glycol, de propylène glycol, le distéaryléther, l'alcool stéarylique, l'alcool béhénylique, l'alcool cétylstéarylique.

La teneur en composé insoluble dans l'eau dans la composition prête à l'emploi est supérieure à 30% en poids de la composition oxydante prête à l'emploi, de préférence est comprise entre 30 et 75 % en poids de la composition oxydante prête à l'emploi, et plus particulièrement est comprise entre 30 et 60 % en poids de la composition oxydante prête à l'emploi.

Ainsi lorsque que le ou les composés insolubles dans l'eau selon l'invention sont solides, la composition est sous forme d'une suspension. Lorsque le ou les composés insolubles dans l'eau selon l'invention sont sous forme liquide, par exemple sous forme d'une phase organique insoluble dans la phase aqueuse, la composition est une émulsion, voire une dispersion.

La composition oxydante prête à l'emploi selon l'invention comprend au moins 20% en poids d'eau par rapport au poids total de la composition, de préférence entre 20 et 78 % et encore plus préférentiellement entre 30 et 60 %.

La composition oxydante prête à l'emploi peut en outre comprendre un ou plusieurs solvants organiques.

Le milieu approprié pour les compositions de l'invention est un milieu cosmétique généralement constitué par de l'eau ou par un mélange d'eau et d'au moins un solvant organique. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le glycérol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition oxydante selon l'invention peut contenir en outre des tensioactifs, des polymères épaississants, des polymères amphiphiles pour stabiliser les dispersions, lorsque la composition se trouve sous forme de dispersion.

Il s'agit des matières premières classiquement utilisées. Notons à titre d'exemple :
- les tensioactifs anioniques, non ioniques, cationiques et amphotères possédant une ou plusieurs chaînes alkylées en C₆ à C₂₂, linéaire ou ramifiée.
- les polymères amphiphiles, comme les copolymères à blocs amphiphiles hydrosolubles ou hydrodispersibles, les polymères associatifs porteurs sur leur chaîne principales de groupements alkyles et/ou aryles en C₆ à C₂₂, oxyéthylénés ou non, téléchéliques ou greffés, ces polymères peuvent être de charge cationique, anionique, amphotère ou non ionique,
- les polymères épaississants comme les polymères acryliques réticulés, les polysaccharides naturels ou modifiés.

La composition oxydante prête à l'emploi selon l'invention peuvent également contenir les additifs traditionnellement utilisés dans le domaine en particulier des agents séquestrants tels l'éthylènediaminetétraacétique, le pentasodium pentétate (dénomination CTFA) ou l'acide étidronique ; des agents stabilisants chimiques du peroxyde d'hydrogène tels les sels de stannate et de pyrophosphate de métaux alcalins (comme le sodium, le potassium par exemple), ou le salicylate de sodium ; des parfums ; des agents anti-mousse ; des polymères substantifs cationiques ou amphotères ; des polymères conditionneurs hydrosolubles ou non, ou des chélatants.

Bien entendu, l'homme de l'art veillera à choisir le ou les éventuels composés complémentaires mentionnés ci-avant, afin que les propriétés de la composition oxydante prête à l'emploi ne soient pas, ou substantiellement pas, altérées par la ou les adjonctions envisagées.

Le pH de la composition oxydante prête à l'emploi selon l'invention est avantageusement compris entre 2 et 12, plus particulièrement entre 2,5 et 7, et de préférence entre 3 et 6.

Le pH peut être ajusté classiquement et si nécessaire par ajout d'agents basifiants, tels que par exemple, l'ammoniaque, la monoéthanolamine, la diéthanolamine, la triéthanolamine, l'isopropanolamine, le 1,3-diamino-propane, un (bi)carbonate alcalin ou d'ammonium, un carbonate organique tel que le carbonate de guanidine, ou bien encore un hydroxyde alcalin, tous ces composés pouvant bien entendu être pris seuls ou en mélange.

Comme précisé précédemment dans l'introduction, la composition prête à l'emploi selon l'invention peut résulter d'un mélange extemporanée d'une ou plusieurs compositions pulvérulentes anhydres avec au moins une composition aqueuse.

Par décoloration, on entend au sens de la présente invention destruction totale ou partielle des pigments naturels présents dans les fibres kératiniques (en particulier eumélanines et phaéomélanines).

La présente invention a de même pour objet un procédé de décoloration de fibres kératiniques humaines, en particulier des cheveux, consistant à appliquer sur la zone des fibres kératiniques humaines, sèches ou humides, à décolorer, la composition oxydante prête à l'emploi selon l'invention ; à la laisser agir pendant un temps de pose suffisant pour obtenir la décoloration recherchée ; à éliminer la composition par un rinçage à l'eau, suivi d'un lavage avec un shampoing, puis éventuellement d'un séchage.

Le temps de pose varie entre 1 et 60 minutes, plus préférentiellement entre 5 et 40 minutes.

Par décapage, on entend au sens de la présente invention destruction totale ou partielle des pigments ou colorants synthétiques présents sur ou dans les fibres kératiniques résultant d'un procédé de coloration directe ou d'oxydation.

La présente invention porte également sur un procédé de décapage de fibres kératiniques humaines, en particulier des cheveux colorés, consistant à appliquer sur la zone des fibres kératiniques humaines colorés, sèches ou humides, à décaper, la composition de décapage prête à l'emploi selon l'invention ; à la laisser agir pendant un temps de pose suffisant pour obtenir le décapage recherché ; à éliminer la composition par un rinçage à l'eau, suivi d'un lavage avec un shampoing, puis éventuellement d'un séchage.

Le temps de pose varie entre 1 et 60 minutes, plus préférentiellement entre 5 et 40 minutes.

Un autre objet de l'invention est un procédé de teinture d'oxydation des fibres kératiniques humaines et en particulier les cheveux mettant en oeuvre une composition colorante comprenant, dans un support approprié pour la teinture des fibres kératiniques, au moins un précurseur de colorant d'oxydation et une composition oxydante prête à l'emploi telle que définie ci-dessus. Au moment de l'emploi, la composition oxydante prête à l'emploi décrite est appliquée sur les fibres kératiniques avant ou après l'application de la composition colorante ou mélangée avec cette composition colorante ; les compositions individuelles ou mélangées sont laissées poser pendant 1 à 60 minutes environ, de préférence 5 à 40 minutes environ, ensuite suit une étape de rinçage, de lavage au shampooing, et éventuellement un rinçage et enfin un séchage. Les compositions individuelles colorante et oxydante décrites peuvent être appliquées dans n'importe quel ordre, avec ou sans rinçage intermédiaire.

La composition colorante mise en oeuvre dans ce procédé de coloration fait intervenir les précurseurs de colorants d'oxydation classiques tels que les bases d'oxydation choisies parmi les phénylènediamines, les bis-phénylalkylènediamines, les para-aminophénols, les ortho-aminophénols, les bases hétérocycliques et leurs sels d'addition ; éventuellement combinés à des coupleurs usuels, comme par exemple les méta-phénylènediamines, les méta-aminphénols, les méta-diphénols, les coupleurs naphtaléniques, les coupleurs hétérocycliques ainsi que leurs sels d'addition.

Un autre objet de l'invention est un procédé de coloration directe éclaircissante des fibres kératiniques humaines et en particulier les cheveux mettant en oeuvre une composition colorante comprenant, dans un support approprié pour la teinture des fibres kératiniques, au moins un colorant direct et une composition oxydante prête à l'emploi telle que définie ci-dessus. Au moment de l'emploi, la composition oxydante prête à l'emploi décrite est appliquée sur les fibres kératiniques avant ou après l'application de la composition colorante ou mélangée avec cette composition colorante; les compositions individuelles ou mélangées sont laissées poser pendant 3 à 40 minutes environ, de préférence 5 à 30 minutes environ, ensuite suit une étape de rinçage, de lavage au shampooing, et éventuellement un rinçage et enfin un séchage. Les compositions individuelles colorante et oxydante décrites peuvent être appliquées dans n'importe quel ordre, avec ou sans rinçage intermédiaire.

Le colorant direct utilisé dans ce procédé de coloration directe éclaircissante est un colorant classique choisi parmi les colorants nitrés de la série benzénique neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

La présente invention a aussi pour objet un procédé de traitement des fibres kératiniques humaines et en particulier les cheveux, en vue d'obtenir une déformation permanente de ces dernières, en particulier sous la forme de cheveux permanentés, ce procédé comprenant les étapes suivantes.

Une composition réductrice est appliquée sur la matière kératinique à traiter, la fibre kératinique étant mise sous tension mécanique avant, pendant ou après ladite application. Celle-ci se fait mèche par mèche ou globalement.

Il convient de manière classique, de laisser reposer pendant quelques minutes, généralement 5 minutes à une heure, la chevelure sur laquelle a été appliquée la composition réductrice. Ainsi, le réducteur a le temps d'agir sur le cheveu. Cette phase d'attente est effectuée de préférence à une température allant de 35°C à 45°C, en protégeant de préférence également les cheveux par un bonnet.

La fibre kératinique imprégnée de la composition réductrice est éventuellement rincée par une composition aqueuse.

La composition oxydante prête à l'emploi de l'invention est appliquée sur la fibre kératinique éventuellement rincée, afin de fixer la nouvelle forme imposée aux cheveux. De nouveau, la chevelure traitée est laissée au repos, pendant 3 à 20 minutes, de préférence entre 5 à 10 minutes.

La fibre kératinique subit, à nouveau, un rinçage, généralement à l'eau.

L'invention a enfin pour objet des dispositifs à plusieurs compartiments, ou « kits », pour la mise en oeuvre des procédés précédemment cités.

Un autre objet de l'invention est un dispositif à 2 compartiments pour la teinture ou pour la déformation permanente ou pour la décoloration des fibres kératiniques en particulier de fibres kératiniques humaines. Un premier compartiment renferme soit une composition de coloration d'oxydation ou directe, soit une composition réductrice, soit une première composition oxydante, comprenant de préférence un sel peroxygéné et un second compartiment renferme la composition oxydante prête à l'emploi définie ci-dessus.

L'exemple suivant illustre l'invention sans présenter un caractère limitatif.

Composition oxydante :

| | |
|---|---|
| Distéaryléther | 35g |
| Laurylsulfate de sodium | 2g |
| Brij 700 | 1g |
| Ammonium acryloyldiméthyl taurate/steareth 25 methacrylate crosspolymer | 2g |
| Stannate de sodium, 6H₂O | 0,04g |
| EDTA | 0,02g |
| Pyrophosphate trétrasodique, 10H₂O | 0,03g |
| Peroxyde d'hydrogène | 6g |
| Acide phosphorique | qsp pH3 |
| Eau déminéralisée | qsp100g |

Cette composition oxydante est mélangée au moment de l'emploi à une poudre décolorante Platine Precision® contenant 50 % de persulfates, 24,1 % de silicates et 2,6 % de chlorure d'ammonium avec un rapport poudre décolorante/composition oxydante égal à 1/1,5. Le mélange obtenu est appliqué sur des mèches de cheveux châtains pendant 40 min. Après rinçage, lavage avec un shampooing standard, nouveau rinçage et séchage, les mèches présentent un très fort pouvoir d'éclaircissement.

Des résultats similaires peuvent être obtenus en remplaçant le distéaryléther poids pour poids par de la poudre Nylon-6 proposée par Induchem sous la dénomination Inducos.

## Revendications

1. Composition oxydante prête à l'emploi destinée au traitement des matières kératiniques, telles que les fibres kératiniques humaines, en particulier les cheveux, comprenant dans un milieu cosmétiquement acceptable :
(a) au moins un agent oxydant,
(b) au moins 30% en poids d'au moins un composé organique oxygéné non colorant insoluble dans l'eau, c'est-à-dire présentant une solubilité dans l'eau à température ambiante (25°C) inférieure à 0,5 % en poids, par rapport au poids total de ladite composition, les composés organiques oxygénés insolubles dans l'eau étant de nature polymérique choisis parmi les polyamides 6, 66, 11, les polyesters, les polyuréthanes, les polycyanoacrylates, les polyméthacrylates de méthyle, les polycarbonates, le téflon, les résines ou élastomères de silicone,
(c) au moins 20% en poids d'eau par rapport au poids total de ladite composition.

2. Composition selon la revendication 1, **caractérisée en ce que** l'agent oxydant est choisi parmi le peroxyde d'hydrogène, le peroxyde d'urée, les bromates de métaux alcalins, les perborates, persulfates, percarbonates, et peroxydes de métaux alcalins ou alcalino-terreux, les peracides ou leurs mélanges ; et les enzymes oxydases.

3. Composition selon la revendication 2, **caractérisée en ce que** l'agent oxydant est le peroxyde d'hydrogène.

4. Composition selon l'une quelconque des revendications 1 à 3, **caractérisée en ce que** la teneur en agent oxydant dans la composition prête à l'emploi est comprise entre 2 et 35 % en poids de la composition prête à l'emploi, de préférence entre 5 et 30 % en poids de la composition prête à l'emploi.

5. Composition selon les revendications précédentes, **caractérisée en ce que** la teneur en composés organiques oxygénés non colorants insolubles dans l'eau dans la composition prête à l'emploi est supérieure à 30% en poids de la composition prête à l'emploi, de préférence est comprise entre 30 et 75 % en poids de la composition prête à l'emploi, et plus particulièrement est comprise entre 30 et 60 % en poids de la composition prête à l'emploi.

6. Composition selon l'une quelconque des revendications précédentes, **caractérisée en ce que** la composition prête à l'emploi comprend de 20 à 78 % en poids d'eau par rapport au poids total de la composition.

7. Composition selon l'une quelconque des revendications précédentes, **caractérisée en ce qu'**elle comprend en outre :
- les tensioactifs anioniques, non ioniques, cationiques et amphotères possédant une ou plusieurs chaînes alkylées en C₆ à C₂₂, linéaire ou ramifiée,
- les polymères amphiphiles, comme les copolymères à blocs amphiphiles hydrosolubles ou hydrodispersibles, les polymères associatifs porteurs sur leur chaîne principales de groupements alkyles et/ou aryles en C₆ à C₂₂, oxyéthylénés ou non, téléchéliques ou greffés, de charge cationique, anionique, amphotère ou non ionique,
- les polymères épaississants comme les polymères acryliques réticulés, les polysaccharides naturels ou modifiés.

8. Composition selon l'une quelconque des revendications précédentes, **caractérisée en ce qu'**elle comprend les additifs choisis parmi des agents séquestrants tels l'éthylènediaminetétraacétique, le pentasodium pentetate ou l'acide étidronique ; des agents stabilisants chimiques du peroxyde d'hydrogène tels les sels de stannate et de pyrophosphate de métaux alcalins, ou le salicylate de sodium ; des parfums ; des agents anti-mousse ; des polymères substantifs cationiques ou amphotères ; des polymères conditionneurs hydrosolubles ou non, ou des chélatants.

9. Composition selon l'une quelconque des revendications précédentes, **caractérisée en ce que** le pH de la composition prête à l'emploi est compris entre 2 et 12, plus particulièrement entre 2,5 et 7, et de préférence entre 3 et 6.

10. Procédé de décoloration de fibres kératiniques humaines, en particulier des cheveux, **caractérisé en ce que** l'on applique sur la zone des fibres kératiniques humaines à décolorer, la composition oxydante prête à l'emploi telle que définie à l'une quelconque des revendications 1 à 9 ; à la laisser agir pendant un temps de pose suffisant pour obtenir la décoloration recherchée ; à éliminer la composition par un rinçage à l'eau, suivi d'un lavage avec un shampoing, puis éventuellement d'un séchage.

11. Procédé de décapage de fibres kératiniques humaines, en particulier des cheveux colorés, **caractérisé en ce que** l'on applique sur la zone des fibres kératiniques humaines colorés à décaper, la composition oxydante prête à l'emploi telle que définie à l'une quelconque des revendications 1 à 9 ; à la laisser agir pendant un temps de pose suffisant pour obtenir le décapage recherché ; à éliminer la composition par un rinçage à l'eau, suivi d'un lavage avec un shampoing, puis éventuellement d'un séchage.

12. Procédé de teinture d'oxydation des fibres kératiniques humaines et en particulier les cheveux, **caractérisé en ce que** l'on applique sur les fibres une composition colorante comprenant, dans un support approprié pour la teinture des fibres kératiniques, au moins un précurseur de colorant d'oxydation et une composition oxydante prête à l'emploi telle que définie à l'une quelconque des revendications 1 à 9, la composition oxydante étant appliquée avant ou après l'application de la composition colorante ou mélangée avec la composition colorante ; les compositions individuelles ou les mélanges sont laissés poser pendant 1 à 60 minutes environ, de préférence 5 à 40 minutes environ, ensuite suit une étape de rinçage, de lavage au shampooing, et suivi éventuellement d'un rinçage et d'un séchage.

13. Procédé de coloration directe éclaircissante des fibres kératiniques humaines et en particulier les cheveux, **caractérisé en ce que** l'on applique sur les fibres une composition colorante comprenant, dans un support approprié pour la teinture des fibres kératiniques, au moins un colorant direct et une composition oxydante prête à l'emploi telle que définie à l'une quelconque des revendications 1 à 9, la composition oxydante étant appliquée sur les fibres kératiniques avant ou après l'application de la composition colorante ou mélangée avec la composition colorante ; les compositions individuelles ou mélangées sont laissées poser pendant 1 à 60 minutes environ, de préférence 5 à 40 minutes environ, ensuite suit une étape de rinçage, de lavage au shampooing, et éventuellement à nouveau un rinçage et enfin un séchage.

14. Procédé de déformation permanente des fibres kératiniques humaines et en particulier les cheveux, comprenant les étapes suivantes :
- on applique une composition réductrice sur la matière kératinique à traiter, la fibre kératinique étant mise sous tension mécanique avant, pendant ou après ladite application,
- on laisse reposer la chevelure sur laquelle a été appliquée la composition réductrice,
- éventuellement, on rince par une composition aqueuse,
- on applique la composition oxydante prête à l'emploi telle que définie à l'une quelconque des revendications 1 à 9 sur la fibre kératinique,
- on laisse reposer la chevelure sur laquelle a été appliquée la composition oxydante, puis
- on rince par une composition aqueuse.

15. Utilisation de la composition oxydante prête telle que définie à l'une quelconque des revendications 1 à 9 pour la teinture ou pour la déformation permanente ou pour la décoloration ou pour l'éclaircissement ou pour la coloration des fibres kératiniques en particulier de fibres kératiniques humaines.

16. Dispositif à 2 compartiments pour la teinture ou pour la déformation permanente ou pour la décoloration des fibres kératiniques en particulier de fibres kératiniques humaines comprenant un premier compartiment renfermant soit une composition de coloration d'oxydation ou directe, soit une composition réductrice, soit une première composition oxydante contenant de préférence un sel peroxygéné, et un second compartiment renfermant la composition oxydante prête telle que définie à l'une quelconque des revendications 1 à 9.
